(19) Europäisches Patentamt
European Patent Office
Office européen des brevets

(11) Publication number : **0 476 865 A1**

(12) **EUROPEAN PATENT APPLICATION**

(21) Application number : **91307908.3**

(22) Date of filing : **29.08.91**

(51) Int. Cl.$^5$ : **G03F 7/039,** G03F 7/075

(30) Priority : **31.08.90 JP 230185/90**

(43) Date of publication of application :
**25.03.92 Bulletin 92/13**

(84) Designated Contracting States :
**DE FR GB IT NL**

(71) Applicant : **WAKO PURE CHEMICAL INDUSTRIES LTD**
**1-2 Doshomachi-3-chome**
**Chuo-ku Osaka (JP)**

(72) Inventor : **Urano, Fumiyoshi**
**16-4-301, Nobitome-1-chome**
**Niiza-shi (JP)**
Inventor : **Nakahata, Masaaki**
**6-53, Tonoyamacho**
**Higashimatsuyama-shi (JP)**
Inventor : **Fujie, Hirotoshi**
**438-9, Kamihiroya Tsurugashimamachi**
**Iruma-gun, Saitama-ken (JP)**
Inventor : **Oono, Keiji**
**15-501, Higashisakado-1-chome**
**Sakado-shi (JP)**

(74) Representative : **Baillie, Iain Cameron et al**
**c/o Ladas & Parry, Altheimer Eck 2**
**W-8000 München 2 (DE)**

(54) **Resist material and process for forming pattern using the same.**

(57) A resist material of chemical amplified type comprising (a) a polymer such as a polymer of 1-methylcycloalkyl 4-ethenylphenoxyacetate and 4-hydroxystyrene, etc., (b) a photo-sensitive compound capable of generating an acid when exposed to light, and (c) a solvent for dissolving both the components (a) and (b) is excellent in heat resistance and adhesiveness to substrates, capable of maintaining stable pattern dimension from exposure to light to heat treatment, and capable of forming patterns using deep ultraviolet light, KrF excimer laser light, etc.

EP 0 476 865 A1

## BACKGROUND OF THE INVENTION

This invention relates to a resist material usable for producing semiconductor elements and the like. More particularly, the present invention relates to a resist material usable for forming positive tone patterns using a light source of 400 nm or less as an exposure energy source, for example, i-line light of 365 nm, deep ultraviolet light of 300 nm or less, KrF excimer laser light of 248.4 nm, ArF excier laser light of 193 nm, electron beams, X-rays, etc.

WIth recent higher density and larger scale integration of semiconductor devices, wavelengths used in exposing devices for minute processing, particularly for lithography become shorter and shorter. Now, KrF excimer laser light (248.4 nm) is studied. But, there have been no photosensitive materials suitable for such a wavelength. For example, there are proposed resist materials comprising a resin having high transmittance for light of near 248.4 nm and a photosensitive compound having a group of the formula:

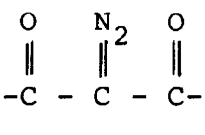

as a resist material for a light source such as KrF excimer laser light and deep UV light (e.g. Japanese Patent Unexamined Publication Nos. 1-80944, 1-154048, 1-155339, etc.). Further, there are developed pattern forming materials comprising a photo-sensitive compound having groups of the formulae:

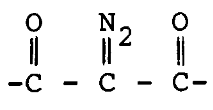 and $-SO_2Cl$

and a resin having high transmittance for light of near 248.4 nm (e.g. Japanese Patent Unexamined Publication No. 1-188852; Y. Tani et al., SPIE's 1989 Sympo., 1086-03, etc.). But these dissolution inhibiting type resist materials have the sensitivity of about 100 to 300 mJ/cm$^2$, so that these materials cannot be used for deep UV light, KrF excimer light, electron beams, etc. which require highly sensitive resist materials.

On the other hand, in order to reduce the exposure energy amount (so as to increase the sensitivity), there are proposed recently chemical amplified resist materials wherein an acid generated by exposure to light is used as a catalyst [H. Ito et al, Polym. Eng. Sci. vol. 23, page 1012 (1983)]. Various chemical amplified resist materials are reported, for example, in U.S. Patent No. 4,491,628 to Ito et al; U.S. Patent No. 4,603,101 to Crivello; W.R. Brunsrold et al: SPIE's 1989 SYmpo., 1086-40; T. Neenan et al: SPIE's 1989 Sympo., 1086-01, etc. But these chemical amplified resist materials have the following disadvantages. When phenol ether type resins such as poly(4-tert-butyoxy-carbonyloxystyrene), poly[(4-tert-butoxycarbonyloxy)-α-methylstyrene], poly(4-tert-butoxystyrene), poly-(4-tert-butoxy-α-methylstyrene) are used, heat resistance is poor, and resist films are easily peeled off at the time of development due to poor adhesion to substrates, resulting in failing to obtain good pattern shapes. Further, when carboxylic acid ester type resins such as poly(tert-buty-4-vinylbenzoate), etc. are used, the transmittance for light of near 248.4 nm is poor due to the aromatic rings of the resin. When poly(tert-butylmethacrylate) and the like is used as the resin, heat resistance and resistance to dry etching are poor.

In order to improve the above-mentioned disadvantages, there are recently proposed various resist materials, for example, a resist material using a copolymer of p-tert-butoxycarboxyloxystyrene and p-hydroxystyrene (European Patent Laid-Open No. 366590), a resist material using a copolymer of p-tetrahydrofuranyloxystyrene and p-hydroxystyrene (European Patent Laid-Open No. 342498), a resist material using a copolymer of p-tert-butoxystyrene and p-hydroxystyrène (Japanese Patent Unexamined Publication No. 2-62544), etc. When patterns are formed by using these resist materials, it is possible to form patterns in an extremely short time, since dimensions of patterns remarkably change with the lapse of time from light exposure to heat treatment. But in practice, since a much time is required from the light exposure to the heat treatment, it is impossible to obtain good patterns in practical production.

As mentioned above, the chemical amplified resist materials have higher sensitivity than known resist materials, but are poor in heat resistance of resin, poor in adhesiveness to substrates, insufficient in transmittance for light of near 248.4 nm, and change the pattern dimension with the lapse of time, resulting in making practical use difficult. Thus, practically usable highly sensitive resist materials overcoming these problems mentioned

above are desired.

SUMMARY OF THE INVENTION

It is an object of the present invention to provide a positive tone resist material having high transmittance for light of deep ultraviolet light, KrF excimer laser light, etc., high sensitivity when exposed to these light sources, electron beams, and X-rays, excellent heat resistance, remarkably excellent adhesiveness to substrates, and being able to obtain patterns with high precision showing no dimensional changes of patterns with the lapse of time, overcoming the disadvantages of the prior art.

The present invention provides a resist material comprising

(a) a polymer represented by the formula:

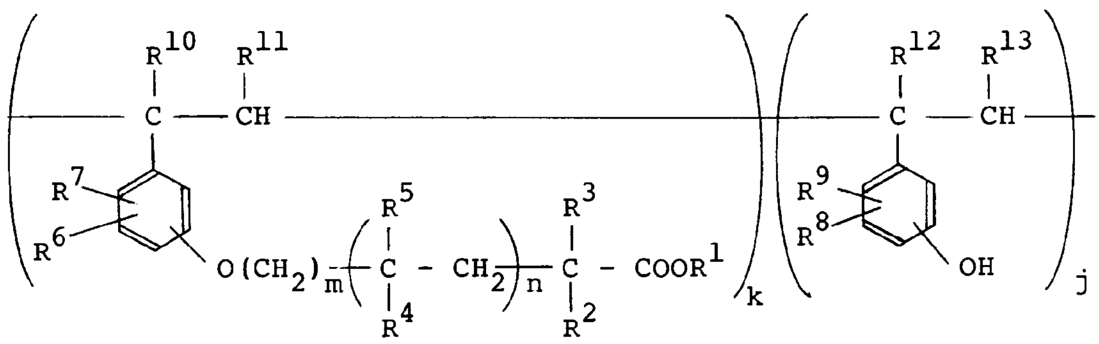

(1)

wherein $R^1$ is a tetrahydropyranyl group, a trimethylsilyl group, a group of the formula:

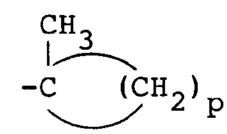

in which p is an integer of 4 or 5, or a group of the formula:

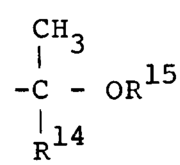

in which $R^{14}$ is a hydrogen atom, a methyl group or an ethyl group; $R^{15}$ is a straight-chain, branched or cyclic alkyl group having 1 to 6 carbon atoms; $R^2$ to $R^5$ are independently a hydrogen atom, or a straight-chain or branched alkyl group having 1 to 8 carbon atoms; $R^6$ to $R^{13}$ are independently a hydrogen atom, or a straight-chain or branched alkyl group having 1 to 6 carbon atoms; k and j are independently natural numbers, provided that $k/k+j = 0.1$ to $0.9$; and m and n are independently zero or an integer of 1 to 3,

(b) a photo-sensitive compound capable of generating an acid when exposed to light, and

(c) a solvent for dissolving both the components (a) and (b).

The present invention also provides a process for forming a pattern which comprises

forming a film on a substrate using the resist material mentioned above,

exposing the film selectively to light,

heating the resulting film,

developing the film to remove the exposed portions, and
forming a photoresist pattern on the unexposed portions of the film.

## BRIEF DESCRIPTION OF THE DRAWINGS

Figs. 1A to 1C are cross-sectional views explaining a pattern forming process using the resist material of the present invention.

Fig. 2 is a graph showing UV spectral curves of resist material film obtained in Example 1 (a full line, before exposure to light; a dotted line, after exposure to light).

Fig. 3 is a cross-sectional view showing a state of overhang during pattern formation in Comparative Example 2 (after 30 minutes).

Fig. 4 is a cross-sectional view showing failure of pattern formation due to strong overhang in Comparative Examples 1 to 4.

## DESCRIPTION OF THE PREFERRED EMBODIMENTS

The chemical amplified resist material of the present invention is characterized by containing a polymer (a) represented by the formula:

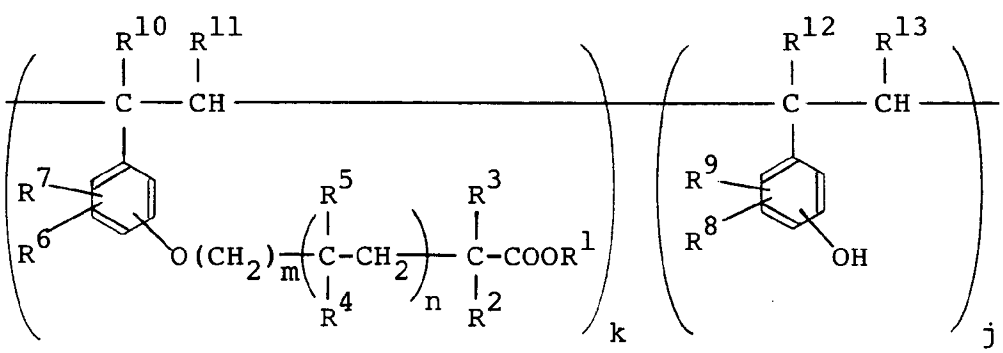

(1)

In the formula (1), $R^1$ is a tetrahydropyranyl group, a trimethylsilyl group, a group of the formula:

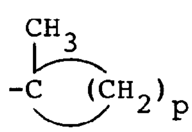

in which p is an integer of 4 or 5, e.g. a 1-methyl-cyclopentyl group or a 1-methylcyclohexyl group, or a group of the formula:

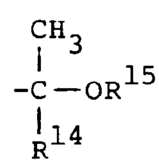

in which $R^{14}$ is a hydrogen atom, a methyl group or an ethyl group; and $R^{15}$ is a straight-chain, branched or cyclic alkyl group having 1 to 6 carbon atoms, e.g. methyl, ethyl, propyl, isopropyl, n-butyl, sec-butyl, tert-butyl, amyl, isoamyl, tert-amyl, hexyl, cyclopropyl, cyclopentyl, cyclohexyl, etc.

$R^2$ to $R^5$ in the formula (1) are independently a hydrogen atom, or a straight-chain or branched alkyl group having 1 to 8 carbon atoms, for exmaple, those exemplified in $R^{15}$ and a cycloheptyl group, a cyclooctyl group, a heptyl group, and an octyl group.

$R^6$ to $R^{13}$ in the formula (1) are independently a hydrogen atom, or a straight-chain or branched alkyl group having 1 to 6 carbon atoms, for example, those exemplified in $R^{15}$.

In the formula (1), k and j are independently natural numbers, provided that $k/k+j = 0.1$ to $0.9$; and m and

n are independently zero or an integer of 1 to 3.

Preferable examples of the polymer of the formula (1) are those represented by the formula:

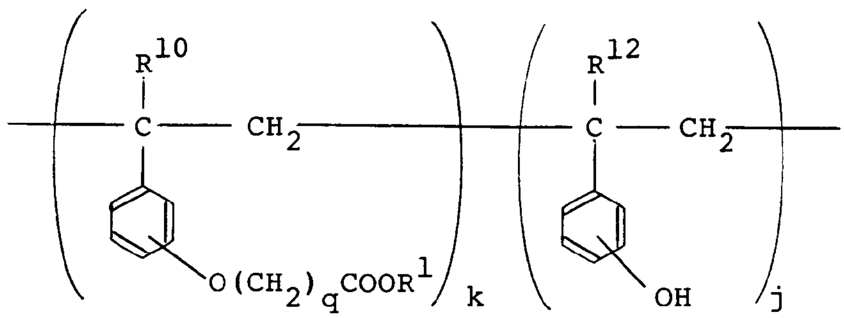

(2)

wherein q is an integer of 1 to 3; and $R^1$, $R^{10}$, $R^{12}$, k and j are as defined above.

Preferable examples of the polymer of the formula (2) are those represented by the formulae:

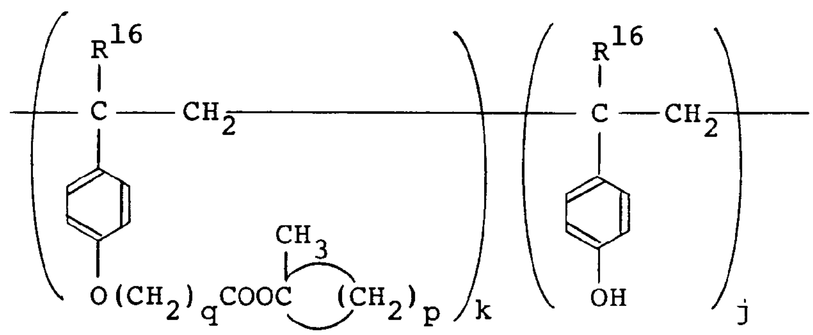

(3)

wherein $R^{16}$ is a hydrogen atom or a methyl group; and k, j, p and q are as defined above, and

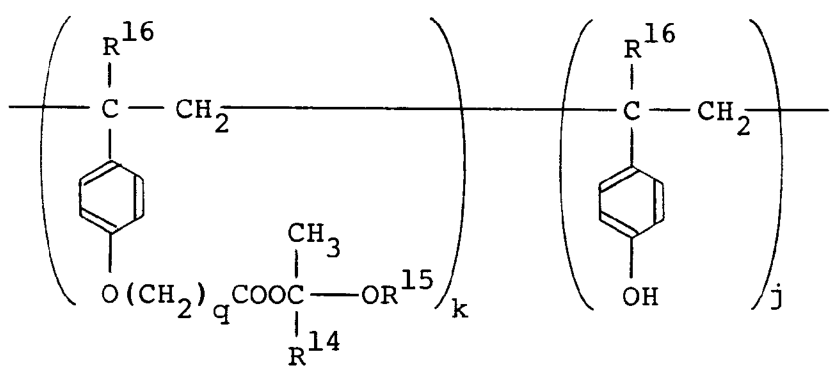

(4)

wherein $R^{14}$ to $R^{16}$, k, j and q are as defined above.

The polymer of the formula (1) is characterized by containing a moiety having a functional group which is easily converted chemically to a carboxylic acid with heating under an atmosphere of an acid generated by exposure to light and represented by the formula:

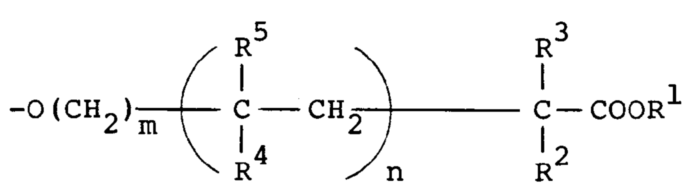

(9)

wherein $R^1$ to $R^5$, m and n are as defined above, that is a monomer component represented by the formula:

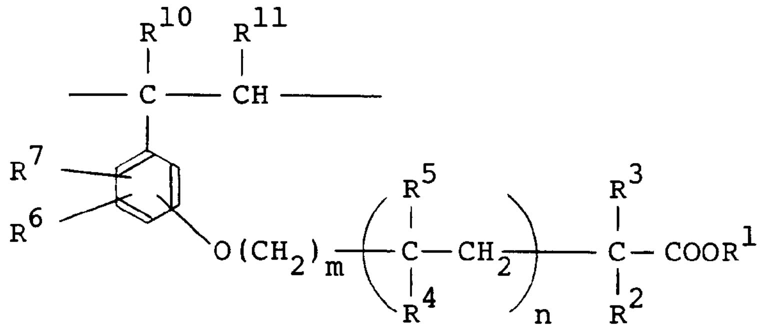

(10)

wherein $R^1$ to $R^7$, $R^{10}$, $R^{11}$, m and n are as defined above.

Examples of monomers containing the monomer component represented by the formula (10) (hereinafter referred to as "monomer having a special functional group") are as follows:

1-methylcyclohexyl 4-ethenylphenoxyacetate,
1-methylcyclopentyl 4-ethenylphenoxyacetate,
1-methylcyclohexyl 4-ethenylphenoxypropionate,
1-cyclohexyloxyethyl 4-ethenylphenoxyacetate,
1-methoxyethyl 4-ethenylphenoxyacetate,
1-ethoxyethyl 4-ethenylphenoxyacetate,
1-methoxy-1-methylethyl 4-ethenylphenoxyacetate,
1-butoxyethyl 4-ethenylphenoxypropionate,
1-isobutyloxyethyl 4-isopropenylphenoxyacetate,
1-ethoxyethyl 3-ethenylphenoxyacetate,
1-methoxy-1-methylethyl 4-isopropenylphenoxy-acetate,
2-tetrahydropyranyl 4-ethenylphenoxyacetate, trimethysilyl 4-ethernylphenoxyacetate, etc.

In the formula (10), when $R^1$ is not the special group as required in the present invention and a straight-chain alkyl group, for example, a compound such as methyl 4-ethenylphenoxyacetate, ethyl 4-ethenylphenoxyacetate, etc., no chemical change takes place even if heated in an atmosphere of acid and no carboxylic acid is generated. On the other hand, when $R^1$ is a branched alkyl group, for example, a compound such as tert-butyl 4-ethenyl-phenoxyacetate, isopropyl 4-ethenylphenoxyacetate, etc., a carboxylic acid can be formed by chemical change when heated under an atmosphere of acid, but the elimination reaction of ester group does not proceed easily and the production of carboxylic acid is small compared with the monomer component of the present invention, resulting in making the difference in dissolution rates of exposed portions and non-exposed portions not so large.

The polymer (a) of the present invention may contain in addition to the monomer component of the formula (10), a monomer component of the formula:

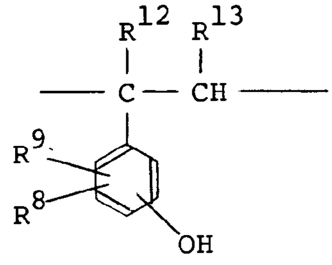

(11)

wherein $R^8$, $R^9$, $R^{12}$ and $R^{13}$ are as defined above.

The monomer component of the formula (11) is derived from a monomer containing a phenolic hydroxyl group. Examples of such a monomer are 4- or 3-hydroxystyrene, 4- or 3-hydroxy-α-methylstyrene, 4-or 3-hydroxy-α-ethylstyrene, 4- or 3-hydroxy-α-n-(or iso-)propylstyrene, etc.

The polymer (a) of the present invention may contain a third component (hereinafter referred to as "third monomer") in addition to the monomer component of the formula (10) and the monomer component of the formula (11) in order to improve transmittance, etc. Examples of the third monomer are methyl methacrylate, hydroxyethyl methacrylate, acrylonitrile, fumaronitrile, styrene, etc.

In the polymer of the present invention, the monomer component of the formula (10) and the monomer component of the formula (11) are preferably contained in a weight ratio of 1:9 to 9:1. But considering the improvement in the heat resistance of the polymer and adhesion to substrate, the weight ratio of 2:8 to 7:3 is more preferable.

Concrete examples of the polymer are as follows:

a polymer of 1-methylcyclohexyl 4-ethenyl-phenoxyacetate and 4-hydroxystryrene,
a polymer of 1-methylcyclopentyl 4-ethenyl-phenoxyacetate and 4-hydroxystyrene,
a polymer of 1-methylcyclohexyl 4-ethenyl-phenoxypropionate and 4-hydroxystyrene,
a polymer of 1-cyclohexyloxyethyl 4-ethenyl-phenoxyacetate and 4-hydroxystyrene,
a polymer of 1-ethoxyethyl 4-ethenylphenoxyacetate and 4-hydroxystyrene,
a polymer of 1-methoxyethyl 4-ethenylphenoxyacetate and 4-hydroxystyrene,
a polymer of 1-methoxy-1-methylethyl 4-ethenylphenoxyacetate and 4-hydroxystyrene,
a polymer of 1-butoxyethyl 4-ethenylphenoxy-propionate and 4-hydroxystyrene,
a polymer of 1-isobutyloxyethyl 4-isopropenyl-phenoxyacetate and 4-hydroxy-α-methylstyrene,
a polymer of 1-ethoxyethyl 3-ethenylphenoxy-acetate and 3-hydroxystyrene,
a polymer of 1-methoxy-1-methylethyl 4-iso-propenylphenoxyacetate and 4-hydroxy-α-methylstyrene,
a polymer of 2-tetrahydropyranyl 4-ethenylphenoxyacetate and 4-hydroxystyrene,
a polymer of trimethylsilyl 4-ethenylphenoxyacetate and 4-hydroxystyrene,
a polymer of 1-ethoxyethyl 4-ethenylphenoxyacetate, 4-hydroxystyrene and fumaronitrile,
a polymer of 1-methylcyclohexyl 4-ethenylphenoxyacetate, 4-hydroxystyrene and methyl methacrylate,

etc.

Considering improvement in sensitivity, resolution, and stable pattern dimension with the lapse of time, the following polymers are more preferable:

a polymer of 1-methylcyclohexyl 4-ethenylphenoxyacetate and 4-hydroxystyrene,
a polymer of 1-methylcyclopentyl 4-ethenylphenoxyacetate and 4-hydroxystyrene,
a polymer of 1-ethoxyethyl 4-ethenylphenoxyacetate and 4-hydroxystyrene,
a polymer of 1-methoxyethyl 4-ethenylphenoxyacetate and 4-hydroxystyrene,
a polymer of 1-methoxy-1-methylethyl 4-ethenylphenoxyacetate and 4-hydroxystyrene,
a polymer of 1-methoxy-1-methylethyl 4-isopropenylphenoxyacetate and 4-hydroxy-α-methylstyrene,
a polymer of 1-ethoxyethyl 4-ethenylphenoxyacetate, 4-hydroxystyrene and fumaronitrile, etc.

When the polymer corresponding to the formula (1) has a straight-chain or branched alkyl group as $R^1$, not the special $R^1$ group as defined in the present invention, for example, a polymer of methyl 4-ethenylphenoxyacetate and 4-hydroxystyrene, a polymer of ethyl 4-ethenylphenoxyacetate and 4-hydroxystyrene, a polymer of tert-butyl 4-ethenylphenoxyacetate and 4-hydroxystyrene, a polymer of isopropyl 4-phenoxyacetate and 4-hydroxystyrene, etc., no chemical change takes place in the presence of an acid generated by exposure to light so as to make pattern formation impossible, or even if the chemical change takes place, it is insufficient and the difference in dissolution rates of exposed portions and non-exposed portions is small, resulting in making the resolution insufficient, causing remarkable dimensional change of pattern from the light exposure to baking with the lapse of time. Thus, such a polymer is remarkably low in performance compared with the polymer used in the present invention, and thus not suitable for practical production.

The polymer of the formula (1) can be produced as follows.

A 4-hydroxystyrene polymer as explained below is reacted with a compound of the formula:

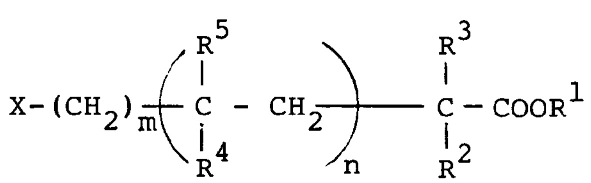 (12)

wherein X is a chlorine atom or an bromine atom; and $R^1$ to $R^5$, m and n are as defined above, in the presence of a base in an organic solvent at 0° to 120°C for 1 to 50 hours, followed by a conventional after-treatment.

As the base, there can be used NaOH, KOH, $Na_2CO_3$, $K_2CO_3$, NaH, sodium methoxide, sodium ethoxide, pyridine, piperidine, morpholine, triethylamine, N-methylpyrrolidine, etc.

As the organic solvent, there can be used ethyl ether, tetrahydrofuran, toluene, acetone, methyl ethyl ketone, ethyl acetate, etc.

The compound of the formula (12) can be produced by reacting a compound of the formula:

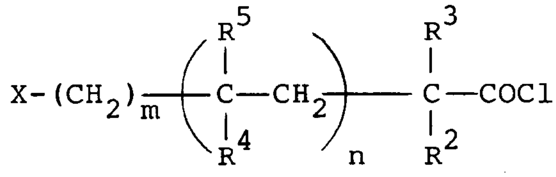 (13)

wherein X, $R^2$ to $R^5$, m and n are as defined above, with a compound of the formula:

$$R^1 - OH \quad (14)$$

wherein $R^1$ is as defined above, in the presence of a base in an organic solvent at 0° to 120°C for 1 to 50 hours, followed by a conventional aftertreatment.

As the base, there can be used NaOH, KOH, NaH, $Na_2CO_2$, $K_2CO_3$, sodium methoxide, sodium ethoxide, pyridine, piperidine, morpholine, triethylamine, N-methylpyrrolidine, etc.

As the organic solvent, there can be used benzene, toluene, dichloromethane, ethyl ether, etc.

The compound of the formula (12) can also be produced by reacting a compound of the formula:

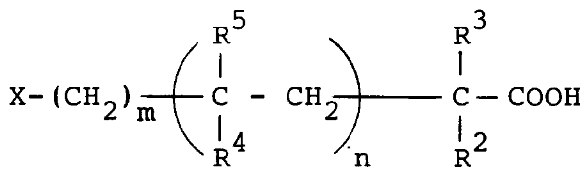 (15)

wherein X, $R^2$ to $R^5$, m and n are as defined above, with a compound of the formula (14), 2-methoxy-1-propylene, ethyl vinyl ether, dihydropyrane, hexamethyldisiloxane or N,O-bis(trimethylsilyl)trifluoroacetamide in the presence or absence of an acid in an organic solvent at -10° to +110°C for 1 to 50 hours, followed by a conventional aftertreatment.

As the acid, there can be used sulfuric acid, phosphoric acid, hydrochloric acid, hydrobromic acid, p-toluenesulfonic acid, etc.

As the organic solvent, there can be used 1,4-dioxane, benzene, toluene, dichloromethane, ethyl ether, etc.

As the 4-hydroxystyrene polymer, there can be used a commercially available one [e.g. MARUKA LYNCUR-M, a trade name, mfd. by Maruzen Petrochemical Co., Ltd.], or those synthesized as mentioned below:

For example, 4-tert-butoxystyrene is polymerized in an organic solvent such as tetrahydrofuran, 1,4-dioxane, benzene, toluene, etc. in the presence of a radical polymerization initiator at 50° to 110°C for 1 to 10 hours in a stream of nitrogen or argon, followed by a conventional aftertreatment to yield 4-tert-butoxystyrene polymer.

As the radical polymerization initiator, there can be used an azo polymerization initiator such as 2,2′-azobisisobutyronitrile, 2,2′-azobis(2,4-dimethylvaleronitrile), 2,2′-azobis(methyl 2-methyl-propionate), or the like, a peroxide polymerization initiator such as benzoyl peroxide, lauroyl peroxide, or the like.

Then, the 4-tert-butoxystyrene polymer is reacted with an acid such as a protonic acid, e.g. sulfuric acid, phosphoric acid, hydrochloric acid, hydrobromic acid, p-toluenesulfonic acid, etc., in an organic solvent such as tetrahydrofuran, acetone, 1,4-dioxane, or the like, at 30 to 110°C for 1 to 20 hours, followed by a conventional aftertreatment to yield the desired 4-hydroxystyrene polymer.

The polymer (a) of the present invention has preferably a weight average molecular weight of about 1,000 to 40,000, more preferably about 3,000 to 20,000.

As the photo-sensitive compound capable of generating an acid when exposed to light (hereinafter referred to as "photoacid generator"), there can be used a photo-sensitive compound which generates an acid when exposed to light and does not influence badly on the photoresist pattern formation. Typical examples of the photoacid generator are compounds of the formulae (5), (6), (7) and (8):

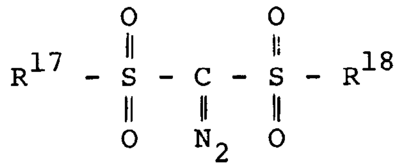

(5)

wherein $R^{17}$ and $R^{18}$ are independently a straight-chain, branched or cyclic alkyl group having 1 to 10 carbon atoms, a straight-chain or branched haloalkyl group having 1 to 10 carbon atoms, or a group of the formula:

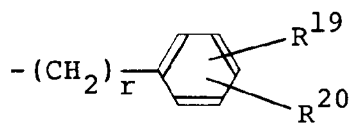

wherein $R^{19}$ and $R^{20}$ are independently a hydrogen atom, a halogen atom, a straight-chain, branched or cyclic alkyl group having 1 to 10 carbon atoms, a straight-chain or branched haloalkyl group having 1 to 10 carbon atoms, a straight-chain or branched alkoxy group having 1 to 10 carbon atoms, a nitro group, or a cyano group; and r is zero or an integer of 1 to 5. Preferable examples of $R^{17}$ and $R^{18}$ are independently a straight-chain, branched or cyclic alkyl group having 1 to 10 carbon atoms, a phenyl group, an alkyl substituted phenyl group, a benzyl group, an alkyl substituted benzyl group, a phenethyl group or an alkyl substituted phenethyl group.

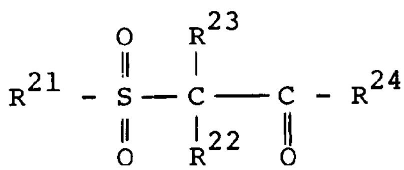

(6)

wherein $R^{21}$ is a straight-chain, branched or cyclic alkyl group having 1 to 10 carbon atoms, a trifluoromethyl group, a phenyl group or a p-tolyl group; $R^{22}$ is a hydrogen atom, a halogen atom, a straight-chain, or branched alkyl group having 1 to 5 carbon atoms, or a straight-chain or branched haloalkyl group having 1 to 5 carbon atoms; $R^{23}$ is a hydrogen atom, a straight-chain or branched alkyl group having 1 to 5 carbon atoms, or a straight-chain or branched haloalkyl group having 1 to 5 carbon atoms; $R^{24}$ is a straight-chain, branched or cyclic alkyl group having 1 to 10 carbon atoms, a phenyl group, a substituted phenyl group (wherein the substituent is a straight-chain or branched alkyl group having 1 to 5 carbon atoms, a straight-chain or branched alkoxy group having 1 to 5 carbon atoms, a halogen atom, or an alkylthio group). Preferable examples are $R^{21}$ and $R^{24}$ being independently a straight-chain, branched or cyclic alkyl group having 1 to 10 carbon atoms, a phenyl group or a p-tolyl group; and $R^{22}$ and $R^{23}$ being methyl groups. Further preferable examples are $R^{21}$ being a straight-chain, branched or cyclic alkyl group having 1 to 6 carbon atoms, a phenyl group or a p-tolyl group; $R^{24}$ being a branched or cyclic alkyl group having 3 to 6 carbon atoms, a phenyl group or a p-tolyl group; and $R^{22}$ and $R^{23}$ being methyl groups.

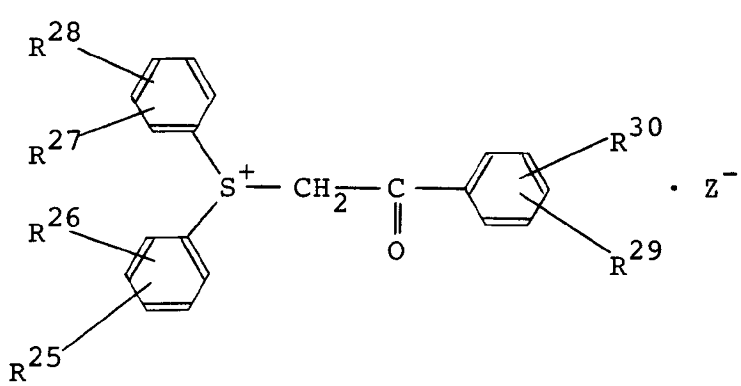

(7)

wherein $R^{25}$ to $R^{30}$ are independently a hydrogen atom, a halogen atom, a straight-chain, branched or cyclic alkyl group having 1 to 10 carbon atoms, a straight-chain or branched alkoxy group having 1 to 10 carbon atoms; and $Z^-$ is a perchlorate ion, a p-toluenesulfonate ion or a trifluoromethanesulfonate ion.

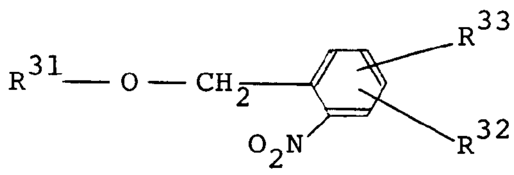

(8)

wherein $R^{31}$ is a trichloroacetyl group, a p-toluenesulfonyl group, a p-trifluoromethylbenzenesulfonyl group, a methanesulfonyl group, or a trifluoromethanesulfonyl group; $R^{32}$ and $R^{33}$ are independently a hydrogen atom, a halogen atom or a nitro group.

Concrete examples of the photoacid generator are as follows.

2,6-dinitrobenzyl p-toluenesulfonate,
2,6-dinitrobenzyl trichloroacetate,
2-nitrobenzyl p-toluenesulfonate,
2-nitrobenzyl trichloroacetate,
2,4-dinitrobenzyl trifluorobenzenesulfonate,
bis(p-toluenesulfonyl)diazomethane,
bis(cyclohexylsulfonyl)diazomethane,
bis(isopropylsulfonyl)diazomethane,
1-p-toluenesulfonyl-1-methanesulfonyldiazomethane,
bis(tert-butylsulfonyl)diazomethane,
1-cyclohexylsulfonyl-1-tert-butylsulfonyldiazomethane,
1-p-toluenesulfonyl-1-cyclohexylsulfonyldiazomethane,
diphenyl-p-methylphenacylsulfonium perchlorate,
diphenyl-2,5-dimethoxyphenacylsulfonium p-toluenesulfonate,
diphenylphenacylsulfonium trifluoromethanesulfonate,
2-methyl-2-p-toluenesulfonylpropiophenone,
2-p-toluenesulfonylpropiophenone,
2-p-toluenesulfonyl-2-cyclohexylcarbonylpropnane,
2,4-dimethyl-2-p-toluenesulfonyl-3-pentanone,
2-benzenesulfonyl-2-cyclohexanecarbonylpropane,
2-cyclohexylsulfonyl-2,4,4-trimethyl-3-pentanone,
2-cyclohexylsulfonyl-2,4,4-trimethyl-3-hexanone,
2,4-dimethyl-4-trifluoromethyl-3-hexanone,
2-benzenesulfonyl-2-(4-methoxybenzoyl)propane,
2-methanesulfonyl-2-methyl-(4-methylthio)-propiophenone, etc.

As the solvent (c), there can be used those which can dissolve both the polymer (a) and the photoacid generator (b). Preferable solvents are those having no absorption at near 365 nm and 248 nm. Concrete examples of the solvents are ethyl Cellosolve acetate, methyl Cellosolve acetate, diethylene glycol dimethyl ether, ethyl lactate, methyl lactate, 1,4-dioxane, propylene glycol monomethyl ether, or ethylene glycol monoisopropyl ether, etc.

Pattern formation using the resist material of the present invention can be carried out, for example, as follows.

The resist material of the present invention is coated on a substrate such as a silicon wafer so as to make the thickness 0.5 to 2 μm (when used as the uppermost layer of three layer laminate, the thickness is about 0.1 to 0.5 μm), and heated at 70° to 130°C for 10 to 30 minutes in an oven, or baked at 70° to 130°C for 1 to 2 minutes on a hot plate. Thus, a mask for forming the desired pattern is formed on the resulting resist film and exposed to deep ultraviolet light of 300 nm or less with the exposure dose of about 1 to 100 mJ/cm$^2$, followed by development using a developing solution such as an aqueous solution of 0.1 to 5% tetramethylammonium hydroxide (TMAH) for 0.5 to 3 minutes by a conventional method such as a dipping method, a puddle method, a spray method, etc., to form the desired pattern on the substrate.

The mixing ratio of the polymer (a) to the photoacid generator (b) in the positive tone resist material is preferably 0.01 to 0.3 part by weight, more preferably 0.01 to 0.1 part by weight, of the photoacid generator (b) per part by weight of the polymer (a).

As the developing solution, there can be used alkaline solutions which can hardly dissolve non-exposed portions and can dissolve exposed portions depending on the solubility for alkaline solution of the resin used in the resist material. The concentration of the alkaline solution is usually 0.01 to 20% by weight.

As the alkaline solution, there can be used solutions of organic amines such as TMAH, choline, triethanolamine, etc.; inorganic alkalis such as NaOH, KOH, etc.

The polymer (a) used in the present invention has no absorption in the wavelength region of 300 nm or more, and has extremely high transmittance for light of i-line light of 365 nm. Further, since the polymer (a) has a hydroxystyrene skeleton, there can be obtained a pattern having good heat resistance, resistance to dry etching, excellent adhesion to the substrate, and dimensional stability with the lapse of time due to the monomer having the special functional group.

The resist material of the present invention can generate an acid when exposed to not only KrF excimer laser light but also i-line light and electron beams, and has a chemical amplifying function. Therefore, the resist material of the present invention is able to form a pattern by applying the chemical amplifying method when exposed to a low exposing amount of deep ultraviolet light, KrF excimer laser light (248.4 nm), i-line light (365 nm), or electron beams.

When the resist material is exposed to KrF excimer light, deep ultraviolet light, i-line light, electron beams or X-rays, the exposed portions generate an acid by the photo reaction as shown by the following chemical equations (I) to (IV):

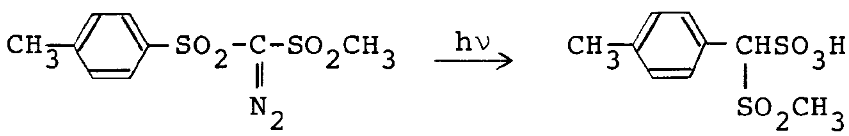


[I]

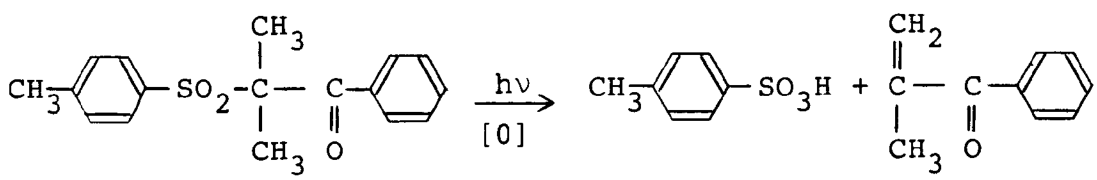


[II]

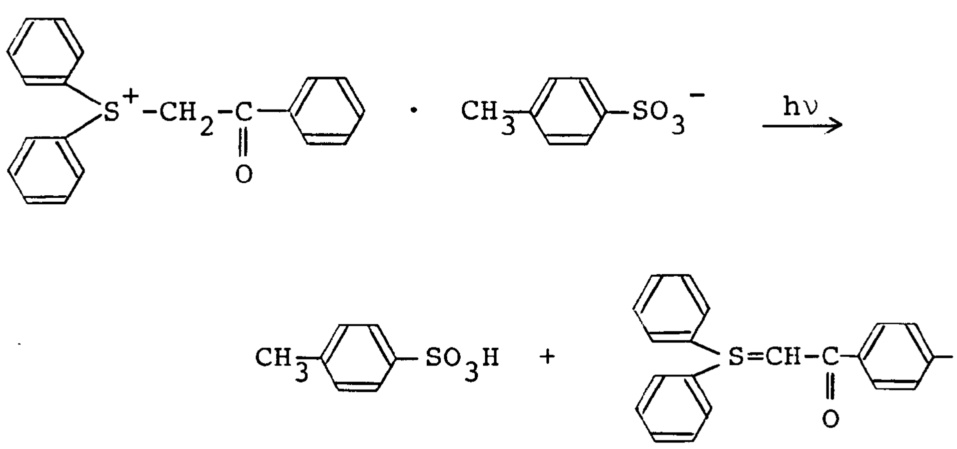


[III]

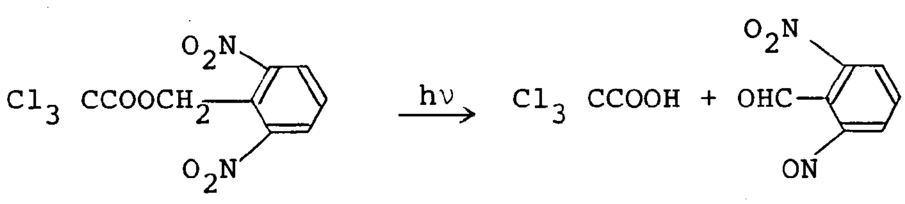


[IV]

When heat treatment is conducted subsequent to the exposure to light, the carboxylic acid ester group of the polymer (a) is suffered from chemical change by the acid according to the chemical equation [V] to produce a carboxylic acid, which is easily soluble in an alkaline solution compound with a phenolic resin. Thus, at the time of development, the carboxylic acid releases into the developing solution.

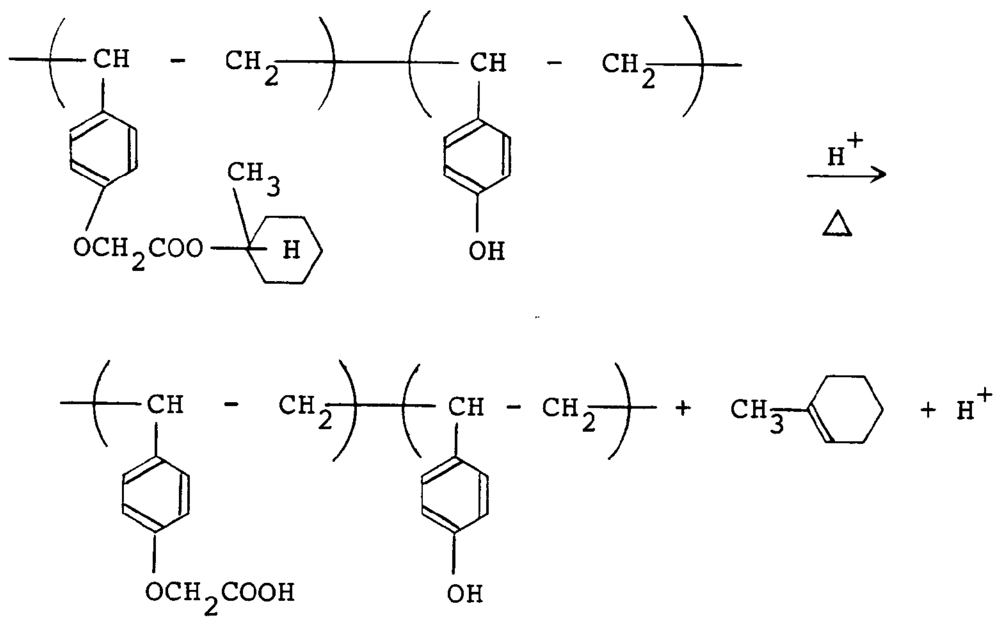

[V]

On the other hand, since non-exposed portions do not generate an acid, no chemical change takes place even if subjected to heat treatment and no alkalisoluble group appears. Further, since the resin per se is high in heat resistance, softening of the resin does not take place at the heat treatment. When the pattern is formed by using the resist material of the present invention, since the carboxylic acid which is an acid stronger than a phenolic hydroxyl group is formed, a larger difference in solubility for the alkaline developing solution takes place between the exposed portions and the non-exposed portions. Further, since the resin in the non-exposed portions is not softened at the time of heat treatment and has strong adhesiveness to the substrate, no peeling of film takes place at the time of development. As a result, there is formed a positive tone pattern having good contrast. In addition, since the acid generated by exposure to light acts catalytically as shown by the equation [V], the exposure to light is only necessary to produce the necessary amount of acid, resulting in making it possible to lower the exposure energy amount.

The present invention is illustrated by way of the following Examples, in which all percents are by weight unless otherwise specified.

Reference Example 1

Synthesis of 2-cyclohexylcarbonyl-2-(p-toluene-sulfonyl)propane

(1) Synthesis of 1-cyclohexyl-2-methyl-1-propanone

To a suspension of magnesium turning (23.9 g, 0.98 atom) in dry ethyl ether, cyclohexyl bromide (160 g, 0.98 mole) was added dropwise under mild reflux, followed by reaction with stirring for 1 hour under reflux. After cooling, the resulting Grignard reagent was added dropwise to a solution of isobutyloyl chloride (95 g, 0.89 mole) in dry ethyl ether at -5 - 0°C, and the resultant mixture was stirred at the same temperature for 3 hours and standed at room temperature overnight. The reaction mixture was poured into $H_2O$, and the organic layer was separated, washed with $H_2O$, dried over anhydrous $MgSO_4$ and evaporated. The residue was distilled under reduced pressure to give 50 g of the title compound as a pale yellow oil having a boiling point of 95 - 100°C/20 mmHg.

$^1$HNMR δppm ($CDCl_3$): 1.06 (6H, d, C<u>H</u>$_3$ x2), 1.12 - 1.87 (10H, m, cyclohexylic C<u>H</u>$_2$ x5), 2.51 (1H, m, cyclohexylic C<u>H</u>), 2.76 (1H, m, C<u>H</u>).

IR(Neat) νcm$^{-1}$: 1710 (C=O).

(2) Synthesis of 2-chloro-1-cyclohexyl-2-methyl-1-propanone

To 1-cyclohexyl-2-methyl-1-propanone (47.6 g, 0.31 mole) obtained in above (1), sulfuryl chloride (42 g, 0.31 mole) was added dropwise at 25 - 35°C, and the mixture was stirred at 50°C for 3.5 hours and then evaporated. The resultant residue was distilled under reduced pressure to give 30.1 g of the title compound as a yellow oil.

bp. 99 - 105°C/18 mmHg.

$^{1}$HNMR δppm ($CDCl_3$): 1.18 - 1.87 (16H, m, C<u>H</u>$_3$ x2 and cyclohexylic C<u>H</u>$_2$ x5), 3.13 (1H, m, cyclohexylic C<u>H</u>).

(3) Synthesis of 2-cyclohexylcarbonyl-2-(p-toluene-sulfonyl)propane

A Solution of 2-chloro-1-cyclohexyl-2-methyl-1-propanone (30.0 g, 0.16 mole) and sodium p-toluene-sulfonate (30.0 g, 0.17 mole) in dimethylsulfoxide was reacted with stirring at 60°C for 20 hours. The reaction mixture was poured into cold $H_2O$, and continued to stirr at 0 - 5°C for 1 hour. The precipitate was filtered, washed with $H_2O$ and dried. The crude solid (18 g) was recrystallized from n-hexane/benzene to give 13.5 g of the title compound as white needles.

m.p. 123 - 123.5°C.

$^{1}$HNMR δppm ($CDCl_3$): 1.19 - 1.91 (16H, m, C<u>H</u>$_3$ x2 and cyclohexylic C<u>H</u>$_2$ x5), 2.45 (3H, s, Ar-C<u>H</u>$_3$), 3.25 (1H, m, cyclohexylic C<u>H</u>), 7.33 (2H, d, J=8Hz, AR 3-H, 5-H), 7.65 (2H, d, J=8Hz, Ar 2-H, 6-H).

IR(KBr-disk) cm$^{-1}$: 1705 (C=O), 1310.

Reference Example 2

Synthesis of 2-cyclohexylsulfonyl-2,4,4-trimethyl-3-pentanone

To cyclohexylthiol (9.3 g, 0.08 mole), an ethanol solution dissolving potassium hydroxide (5.5 g) in ethanol (22 ml) was added dropwise and mixture was reacted with stirring at 30 ± 5°C for 30 minutes. Then 2-chloro-2,4,4-trimethyl-3-pentanone (13.0 g, 0.08 mole) obtained in the same manner as described in Reference Example 1(2) using 2,2,4-trimethyl-3-pentanone was added dropwise to this mixture at 0 - 10°C, reacted with stirring at the same temperature for 3 hours, then at room temperature for 2 hours and standed at room temperature overnight. The reaction mixture was diluted with ethanol (50 ml), added with sodium tungstate (0.4 g), then 30% hydrogen oxide (22.8 g, 0.23 mole) was added dropwise to this solution at 45 - 50°C, and reacted with stirring for 10 hours at the same temperature. After standing at room temperature overnight, the mixture was poured into $H_2O$ (500 ml), extracted with ethyl acetate (100 ml x 3), the organic layer was washed with $H_2O$, dried, evaporated and resultant residue (21 g) was recrystallized from 60% ethanol aqueous solution to give the title compound as white leaflets.

m.p. 56 - 58.5°C.

$^{1}$ HNMR δppm ($CDCl_3$): 1.13 - 2.11 (25H, m, C<u>H</u>$_3$ x5 and cyclohexylic C<u>H</u>$_2$ x5), 3.48 - 3.62 (1H, m, cyclohexylic C<u>H</u>)

IR(KBr-disk) cm$^{-1}$: 1705 (C=O), 1305 ($SO_2$).

Reference Example 3

Synthesis of 2,4-dimethyl-2-(p-toluenesulfonyl)-3-pentanone

Using diisopropylketone in place of 1-cyclohexyl-2-methyl-1-propanone, the reaction was carried out in the same manner as described in Reference Example 1(2) and (3). The crude solid was recrystallized from n-hexane/benzene to afford 2,4-dimethyl-2-(p-toluene-sulfonyl)-3-pentanone as white leaflets.

m.p. 76 - 79°C.

$^{1}$HNMR δppm ($CDCl_3$): 1.15 (6H, d, C<u>H</u>$_3$x2), 1.55 (6H, s, C<u>H</u>$_3$x2), 2.45 (3H, s, Ar-C<u>H</u>$_3$), 3.54 (1H, m, J=7Hz, C<u>H</u>), 7.34 (2H, d, J=8Hz, Ar 3-H, 5-H), 7.65 (2H, d, J=8Hz, Ar 2-H, 6-H).

IR(KBr-disk) νcm$^{-1}$: 1715 (C=O), 1305, 1290.

Reference Example 4

Synthesis of 2-methyl-2-(p-toluenesulfonyl)-propiophenone

Using isobutyrophenone in place of 1-cyclohexyl-2-methyl-1-propanone, the reaction was carried out in the same manner as described in Reference Example 1(2) and (3), the crude solid was recrystallized from methanol to afford the title compound as white needles.

m.p. 64 - 64.5°C.

$^1$HNMR δppm ($CDCl_3$): 1.70 (6H, s, C$\underline{H}_3$ x 2), 2.45 (3H, s, Ar-C$\underline{H}_3$), 7.32 (2H, d, J=7Hz, p-Me-Ar 3-H, 5-H), 7.44 (2H, t, J=7Hz, Ar 3-H, 5-H), 7.54 (1H, t, J=7Hz, Ar 4-H), 7.67 (2H, d, J=7Hz, p-Me-Ar 2-H, 6-H), 7.95 (2H, d, J=7Hz, Ar 2-H, 6-H).

IR(KBr-disk) $cm^{-1}$: 1680 (C=O), 1303, 1290.

Reference Example 5

Synthesis of bis(p-toluenesulfonyl)diazomethane

(1) Synthesis of p-toluenesulfonylazide

After dissolving sodium azide (22.5 g, 0.35 mole) in a small amount of $H_2O$, the resulting solution was diluted with a 90% ethanol aqueous solution (130 ml). To this, an ethanol solution dissolving p-toluenesulfonyl chloride (60 g, 0.32 mole) was added dropwise at 10 - 25°C, followed by reaction at room temperature for 2.5 hours. The reaction solution was concentrated at room temperature under reduced pressure. The resulting oil residue was washed with $H_2O$ several times and dried over anhydrous $MgSO_4$. After removing the drying agent by filtration, there was obtained 50.7 g of the title compound as colorless oil.

$^1$HNMR δppm ($CDCl_3$): 2.43 (3H, s, C$\underline{H}_3$), 7.24 (2H, d, J=8Hz, Ar 3-H, 5-H), 7.67 (2H, d, J=8Hz, AR 2-H, 6-H).

IR(Neat) $\nu cm^{-1}$: 2120 ($N_3$).

(2) Synthesis of bis(p-toluenesulfonyl)methane

To p-thiocresol (20 g, 0.16 mole), an 80% ethanol aqueous solution (75 ml) dissolving potassium hydroxide (10.7 g, 0.16 mole) was added dropwise at room temperature and mixture was stirred at 30 ± 5°C for 30 minutes. Then methylene chloride (13.7 g, 0.16 mole) was added to this mixture and reacted with stirring at 50 ± 5°C for 6 hours. After standing at room temperature overnight, the reaction mixture was diluted with ethanol (30 ml) and $H_2O$ (30 ml), and then added with sodium tungstate (0.6 g). Then 30% hydrogen peroxide (75 g) was added dropwise to this solution at 45 - 50°C, reacted with stirring for 4 hours at the same temperature, then added with $H_2O$ (1.2 ℓ) and standed overnight at room temperature. The precipitate was filtered, washed with $H_2O$ and dried. The resulting solid was recrystallized from ethanol to give 16.5 g of the title compound as white needles.

m.p. 125 - 128°C.

$^1$HNMR δppm ($CDCl_3$): 2.47 (6H, s, C$\underline{H}_3$ x 2), 4.69 (2H, s, C$\underline{H}_2$), 7.37 (4H, d, J=8Hz, Ar 3-H, 5-H x 2), 7.83 (4H, d, J=8Hz, Ar 2-H, 6-H x 2).

IR(KBr-disk) $\nu cm^{-1}$: 1310 ($SO_2$).

(3) Synthesis of bis(p-toluenesulfonyl)diazomethane

To a solution of sodium hydroxide (1.5 g) in a 60% ethanol aqueous solution (70 ml), bis(p-toluenesulfonyl)methane (10.0 g, 0.03 mole) obtained in above (2) was added, then an ethanol solution of p-toluenesulfonylazide (7.3 g, 0.037 mole) obtained in above (1) was added dropwise at 5 - 10°C, followed by the reaction at room temperature for 7 hours. After standing at room temperature overnight, the precipitate was filtered, washed with ethanol and dried. The resultant solid (5.5 g) was recrystallized from ethanol to give 3.5 g of the title compound as pale yellow leaflets.

m.p. 121.5 - 123°C.

$^1$HNMR δppm ($CDCl_3$): 2.46 (6H, s, C$\underline{H}_3$ x 2), 7.36 (4H, d, J=8Hz, (Ar 3-H, 5-H) x 2), 7.87 (4H, d, J=8Hz, (Ar 2-H, 6-H) x 2).

IR(KBr-disk) $\nu cm^{-1}$: 2110 ($CN_2$), 1345 ($SO_2$).

Reference Example 6

Synthesis of bis(tert-butylsulfonyl)diazomethane

Using tert-butylthiol in place of p-thiocresol, the reaction was carried out in the same manner as described in Reference Example 5. The resutlant crude solid was recrystallized from ethanol to give the title compound as pale yellow needles.

m.p. 121 - 121.5°C.

$^1$HNMR δppm ($CDCl_3$): 1.52 (18H, s, C<u>H</u>$_3$ x 6).

IR(KBr-disk) $\nu cm^{-1}$: 2120 ($CN_2$), 1330 ($SO_2$), 1315.

Reference Example 7

Synthesis of bis(isopropylsulfonyl)diazomethane

Using isopropylthiol in place of p-thiocresol, the synthesis was carried out in the same manner as described in Reference Example 5. The resultant crude solid was recrystallized from methanol to give the title compound as pale yellow needles.

m.p. 82 - 84°C.

$^1$HNMR δppm ($CDCl_3$): 1.46 (12H, d, J=7Hz, C<u>H</u>$_3$ x 4), 3.74 (2H, m, J=7Hz, C<u>H</u> x 2).

IR(KBr-disk) $\nu cm^{-1}$: 2120 ($CN_2$), 1340 ($SO_2$), 1320.

Reference Example 8

Synthesis of 2-nitrobenzyl trichloroacetate

To a solution of trichloroacetic acid (5 g, 0.031 mole), o-nitrobenzylalcohol (9.4 g, 0.061 mole) and pyridine (4.9 g) in methylene chloride (30 ml), N,N'-cyclohexylcarbodiimide (6.3 g, 0.031 mole) was added in a small portion at 5 - 10°C, reacted with stirring at room temperature for 7 hours, and then standed at room temperature overnight. The precipitate was filtered off, conc. hydrochloric acid (30 ml), $H_2O$ (35 ml) and methylene chloride (150 ml) was added to the filtrate, followed by stirring. The organic layer was separated, washed with $H_2O$ (200 ml x 3), dried over anhydrous $MgSO_4$ and evaporated. The resultant residue was chromatographed on silica gel (Wako Gel C-200, a trade name, mfd. by Wako Pure Chemical Industries, Ltd.) with ethyl acetate/n-hexane (1/30 → 1/10) as eluent to afford 6.8 g of the title compound as a pale yellow oil.

$^1$HNMR δppm ($CDCl_3$): 5.81 (2H, s, C<u>H</u>$_2$), 7.51 - 7.76 (3H, m, Ar 4-H, 5-H, 6-H), 8.20 (1H, d, J= 8Hz, Ar 3-H).

IR(Neat) $\nu cm^{-1}$: 1760 (C=O), 1520 ($NO_2$), 1335 ($NO_2$).

Synthesis Example 1

Synthesis of 1-methylcyclohexyl monochloroacetate

To a solution of 1-methylcyclohexan-1-ol (70.8 g, 0.62 mole) and pyridine (8.5 g) in methylene chloride (300 ml), chloroacetylchloride (70 g, 0.62 mole) was added dropwise at 20 - 30°C, reacted with stirring for 2 hours at the same temperature. The precipitate was filtered off and the filtrate was evaporated. The residue (116 g) was chromatographed on silica gel (Wakogel C-200, a trade name, mfd. by Wako Pure Chemical Industries, Ltd.) with n-hexane/ethyl acetate (10/1 v/v) as eluent to give 53.2 g of 1-methylcyclohexyl monochloroacetate as a pale yellow oil.

$^1$HNMR δppm($CDCl_3$): 1.27 - 2.18 (13H, m, C<u>H</u>$_3$ and cyclohexylic proton), 4.00 (2H, s, C<u>H</u>$_2$).

IR $\nu cm_{-1}$(Neat): 1760 (C=O).

Synthesis Example 2

Synthesis of 1-ethoxyethyl monobromoacetate

To a solution of monobromoacetic acid (25 g, 0.18 mole) and a few drops of conc. sulfuric acid in toluene (35 ml), ethyl vinyl ether (23 g, 0.32 mole) was added dropwise at 15 - 25°C, and reacted with stirring at room

temperature for 5 hours. After standing at room temperature overnight, saturated sodium bicarbonate aqueous solution (400 ml) and ethyl acetate (200 ml) were added to the resultant mixture, followed by stirring for 1 hour. The organic layer was separated, washed twice with brine and dried over anhydrous $MgSO_4$. After removing the drying agent by filtration, the filtrate was evaporated and the residue (29 g) was distilled under reduced pressure to give 23.5 g of 1-ethoxyethyl monobromoacetate as a colorless oil.

b.p. 61 - 63°C/4 mmHg.

$^1$HNMR δppm ($CDCl_3$): 1.23 (3H, t, $-CH_2C\underline{H_3}$), 1.44 (3H, d, $-CHC\underline{H_3}$), 3.52 - 3.80 (2H, m, $-C\underline{H_2}CH_3$), 3.84 (2H, s, $BrC\underline{H_2}-$), 5.98 (1H, q, $-C\underline{H}-O-$).

IR νcm$^{-1}$ (Neat): 1725 (C=O).

Synthesis Example 3

Synthesis of 2-tetrahydropyranyl monobromoacetate

Using monobromoacetic acid (25 g, 0.18 mole) and dihydropyran (27.2 g, 0.32 mole), the reaction was carried out in the same manner as described in Synthesis Example 2. The resultant residue (42.5 g) was chromatographed on silica gel (Wako Gel C-200) with n-hexane/ethyl acetate/diethylamine (10/1/0.5 V/V) to afford 25.6 g of 2-tetrahydropyrany1 monobromoacetate as a colorless oil.

$^1$HNMR δppm ($CDCl_3$) : 1.39 - 1.96 (6H, m, THP 3-H, 3-H, 4-H, 4-H, 5-H, 5-H), 3.67 - 4.06 (4H, m, $BrC\underline{H_2}-$ and THP 6-H, 6-H), 6.06 (1H, bs, THP 2-H).

IR νcm$^{-1}$ (Neat) : 1730 (C=O)

Synthesis Example 4

Synthesis of 1-methylcyclopentyl monochloroacetate

Using 1-methylcyclopentan-1-ol (23.0 g, 0.23 mole) and chloroacetylchloride (24.8 g, 0.22 mole), the reaction was carried out in the same manner as described in Synthesis Example 1 to give 22.5 g of 1-methylcyclopentyl monochloroacetate as a colorless oil.

$^1$HNMR δppm ($CDCl_3$) : 1.58 - 2.30 (11H, m, $C\underline{H_3}$ and cyclopentylic proton), 3.97 (2H, s, $ClC\underline{H_2}-$).
IR νcm$^{-1}$ (Neat) : 1755 (C=O).

Synthesis Example 5

Synthesis of 1-methylcyclohexyl monochloropropionate

Using 1-methylcyclohexan-1-ol (26.3 g, 0.23 mole) and monochloropropionyl chloride (27.9 g, 0.22 mole), the reaction was carried out in the same manner as described in Synthesis Example 1 to give 23.0 g of 1-methylcyclohexyl monochloroprpionate as a colorless oil. $^1$HNMR δppm ($CDCl_3$) : 1.17 - 2.21 (13H, m, $C\underline{H_3}$ and cyclohexylic proton), 2.74 (2H, t, J=6.6Hz, $ClCH_2\ C\underline{H_2}COO-$), 3.74 (2H, t, J=6.6Hz, $ClC\underline{H_2}CH_2COO-$). IR νcm$^{-1}$ (Neat) : 1725 (C=O).

Synthesis Example 6

Synthesis of trimethylsilyl monochloroacetate

To a solution of monochloroacetic acid (16.7 g, 0.18 mole) and hexamethyldisiloxane (57.3 g, 0.35 mole)

in toluene (50 ml), conc. sulfuric acid (1 ml) was added and reacted with stirring for 28 hours under reflux. The reaction mixture was evaporated and the residue was distilled under reduced pressure to afford 16.5 g of trimethylsilyl monochloroacetate as a colorless oil.

b.p. 84 - 86°C/60 mmHg.

$^1$HNMR δppm ($CDCl_3$) : 0.28 (9H, s, $C\underline{H}_3$ x 3), 4.37 (2H, s, $ClC\underline{H}_2$).

IR $\nu cm^{-1}$ (Neat) : 1735 (C=O).

Synthesis Example 7

Synthesis of poly(1-methylcyclohexyl 4-ethenyl-phenoxyacetate-4-hydroxystyrene)

(1) Free radical polymerization of 4-tert-butoxystyrene

A solution of 4-tert-butoxyethyrene (17.6 g) in toluene containing catalytic amount of 2,2′-azobisisobutyronitrile (AIBN) was heated at 80°C for 6 hours under nitrogen. After cooling, the reaction mixture was poured into methanol and the polymer was precipitated. The polymer was filtered, washed with methanol and dried under reduced pressure to afford 15.5 g of poly(4-tert-butoxystyrene) as a white powder having $\overline{Mw}$ 10000 (GPC with polystyrene calibration).

(2) Synthesis of poly(4-hydroxystyrene)

A solution of poly(4-tert-butoxystyrene) (15.0 g) obtained in above (1) and hydrochloric acid (10 ml) in 1,4-dioxane was refluxed for 4 hours with stirring. The mixture was cooled, poured into $H_2O$ and the white solid was precipitated. The polymer was filtered, washed with $H_2O$ and dried under reduced pressure to afford 9.7 g of poly(4-hydroxystyrene) as a white powder.

(3) Synthesis of poly(1-methylcyclohexyl 4-ethenyl-phenoxyacetate-4-hydroxystyrene)

To a solution of poly(4-hydroxystyrene) (4.0 g) obtained in above (2) and 1-methylcyclohexyl monochloroacetate (3.2 g) obtained in Synthesis Example 1 in acetone (35 ml), potassium carbonate (2.4 g) and potassium iodide (0.33 g) were added, and refluxed for 6 hours with stirring. After cooling, the precipitate was filtered off, the filtrate was poured into $H_2O$, and the resultant white solid was filtered, washed with $H_2O$ and dried under reduced pressure to afford 4.9 g of the title polymer as a white powder having $\overline{Mw}$ 10000 (GPC with polystyrene calibration). The composition of the polymer was found to be 1-methylcyclohexyl 4-ethenyl phenoxyacetate unit and 4-hydroxystyrene unit in molar ratio of ca. 1:1 based on $^1$HNMR analysis.

Synthesis Examples 8 - 13

Using poly(4-hydroxystyrene) (4.0 g) obtained in the same manner as described in Synthesis Example 7(2) and each monohalogenoacetic ester obtained in Synthesis Examples 2 - 6 or tert-butyl monochloroacetate obtained as a commercial grade, the reaction was carried out in the same manner as described in Synthesis Example 7(3) to afford corresponding polymer.

The results are shown in Table 1.

In Table 1, the ratio of k and j was found by $^1$HNMR analysis, and $\overline{Mw}$ of polymers each and all was found to be ca. 10000 by GPC measurement with polystyrene calibration.

Table 1

| Synthesis Example No. | Acetic acid ester / Using amount | Synthesized polymer | Appearance/yield | k/j |
|---|---|---|---|---|
| 8 | 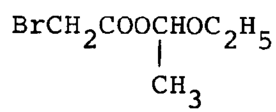 /2.8 g | 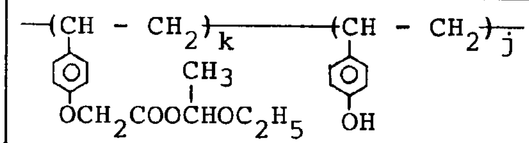 | White powdery crystals /4.4 g | 4/6 |
| 9 | 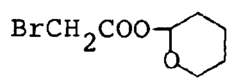 /4.5 g | 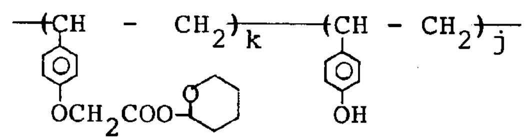 | do. /5.4 g | 6/4 |
| 10 | 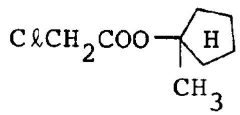 /2.9 g | 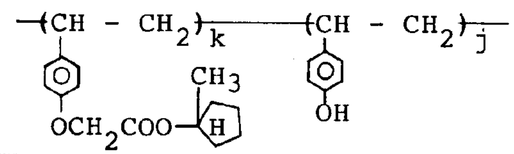 | do. /5.3 g | 1/1 |
| 11 | 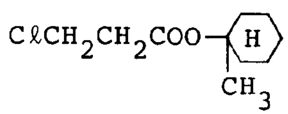 /2.7 g | 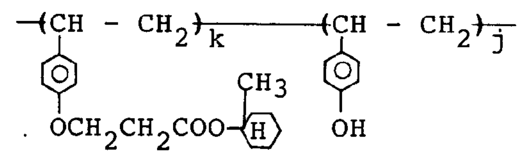 | do. /5.3 g | 4/6 |
| 12 | $C\ell CH_2COOSi(CH_3)_3$ /2.8 g | 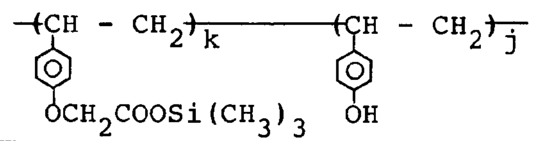 | do. /4.6 g | 1/1 |
| 13 | $C\ell CH_2COOC(CH_3)_3$ /2.6 g | 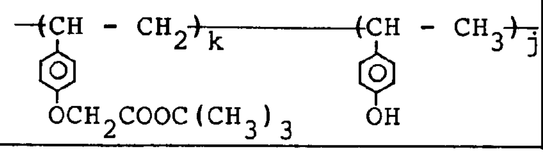 | do. /5.0 g | 1/1 |

Example 1

A Resist material having the following composition was prepared.

| | |
|---|---|
| Poly(1-methylcyclohexyl 4-ethenylphenoxyacetate-4-hydroxystyrene) (Polymer of Synthesis Example 7(3)] | 6.0 g |
| 2-(Cyclohexylcarbonyl)-2-(p-toluenesulfonyl)propane (photoacid generator of Reference Example 1) | 0.3 g |
| Diethylene glycol dimethyl ether | 13.7 g |

Pattern formation using the above-mentioned resist material is explained referring to Fig. 1. The above-mentioned resist material 2 was spin coated on a substrate 1 such as a semiconductor. After soft baking at 90°C for 90 seconds on a hot plate, a resist material film of 1.0 μm thick was obtained (Fig. 1A). Then, the resist material film was selectively exposed to KrF excimer laser light 3 of 248.4 nm rai a mask 4 (Fig. 1B). After baking at 110°C for 90 seconds on a hot plate, development was carried out for 60 seconds using an alkaline developing solution (a 2.38% aqueous solution of tetramethylammonium hydroxide) to remove only exposed portions of the resist material 2 by dissolution. As a result, a positive tone (resin) pattern 2a was obtained (Fig. 1C).

UV Spectral curves of resist material film (1 μm) before and after exposure are shown in Fig. 2. As shown in Fig. 2, the transmittance before and after exposure to light is hardly changed and shows a transmittance of as high as about 65% after exposure to light. The positive tone pattern had an aspect ratio of about 87°, resolution of 0.3 μm lines and spaces, and an exposure energy amount of 15 mJ/$cm^2$.

Example 2

Using the same resist material as used in Example 1, pattern formation was carried out in the same manner as described in Example 1. In this Example, pattern formation was carried out by changing the time from the light exposure to the baking as shown in Table 2. The results are shown in Table 2.

Table 2

| Standing time from light exposure to baking | 15 min | 30 min | 1 hour | 3 hours |
|---|---|---|---|---|
| Resolution of pattern | Good | Good | Good | Good |

Example 3

A resist material having the following composition was prepared and subjected to the same experiments as described in Examples 1 and 2.

| | |
|---|---|
| Poly(1-methylcyclohexyl 4-ethenylphenoxyacetate-4-hydroxystyrene) [Polymer of Synthesis Example 7(3)] | 6.0 g |
| Bis(tert-butylsulfonyl)diazomethane (photoacid generator of Reference Example 6) | 0.5 g |
| Diethylene glycol dimethyl ether | 13.5 g |

As a result, the same good results as obtained in Examples 1 and 2 were obtained. Particularly, even the time from the light exposure to the baking elapsed 5 hours, the pattern dimension was not changed. A positive tone pattern using this resist material had an exposure energy amount of 18 mJ/cm$^2$.

Example 4

A resist material having the following composition was prepared and subjected to the same experiments as described in Examples 1 and 2.

| | |
|---|---|
| Poly(1-methylcyclopentyl 4-ethenylphenoxyacetate-4-hydroxystyrene) [Polymer of Synthesis Example 10] | 6.0 g |
| 2-Cyclohexylsulfonyl-2,4,4-trimethyl-3-pentanone (photoacid generator of Reference Example 2) | 0.6 g |
| Diethylene glycol dimethyl ether | 13.4 g |

As a result, the same good results as obtained in Examples 1 and 2 were obtained. A positive tone pattern was formed by using this resist material and showed an exposure energy amount of 10 mJ/cm$^2$. Further, even the time from the light exposure to the baking elapsed 5 hours, the pattern dimension was not changed.

Example 5

A resist material having the following composition was prepared and subjected to the same experiments as described in Examples 1 and 2.

| | |
|---|---|
| Poly(1-ethoxyethyl 4-ethenylphenoxyacetate-4-hydroxystyrene) [Polymer of Synthesis Example 8] | 6.0 g |
| 2,4-Dimethyl-2-p-toluenesulfonyl-3-pentanone (photoacid generator of Reference Example 3) | 0.3 g |
| Propylene glycol monomethyl ether | 13.7 g |

As a result, the same good results as obtained in Examples 1 and 2 were obtained. A positive tone pattern was formed by using this resist material and showed an exposure energy amount of 8 mJ/cm$^2$.

Example 6

A resist material having the following composition was prepared and subjected to the same experiments as described in Examples 1 and 2.

| | |
|---|---|
| Poly(2-tetrahydropyranyl 4-ethenylphenoxyacetate-4-hydroxystyrene) [Polymer of Synthesis Example 9] | 6.0 g |
| 2-Methyl-2-p-toluenesulfonylpropiophenone (photoacid generator of Reference Example 4) | 0.3 g |
| Diethylene glycol dimethyl ether | 13.7 g |

As a result, the same good results as obtained in Examples 1 and 2 were obtained. A positive tone pattern was formed by using this resist material and showed an exposure energy amount of 15 mJ/$cm^2$.

Example 7

A resist material having the following composition was prepared and subjected to the same experiments as described in Examples 1 and 2.

| | |
|---|---|
| Poly(trimethylsilyl 4-ethenylphenoxyacetate-4-hydroxystyrene) [Polymer of Synthesis Example 12] | 6.0 g |
| Bis(p-toluenesulfonyl)diazomethane (photoacid generator of Reference Example 5) | 0.3 g |
| Ethyl lactate | 13.7 g |

As a result, the same good results as obtained in Examples 1 and 2 were obtained. A positive tone pattern was formed by using this resist material and showed an exposure energy amount of 20 mJ/$cm^2$.

Example 8

A resist material having the following composition was prepared and subjected to the same experiments as described in Examples 1 and 2.

| | |
|---|---|
| Poly(1-methylcyclohexyl 4-ethenylphenoxypropionate-4-hydroxystyrene) [Polymer of Synthesis Example 11] | 6.0 g |
| 2-Methyl-2-p-toluenesulfonylpropiophenone (photoacid generator of Reference Example 4) | 0.3 g |
| Ethyl Cellosolve acetate | 13.7 g |

As a result, the same good results as obtained in Examples 1 and 2 were obtained. A positive tone pattern was formed by using this resist material and showed an exposure energy amount of 25 mJ/$cm^2$.

Example 9

A resist material having the following composition was prepared and subjected to the same experiments as described in Examples 1 and 2.

| | |
|---|---|
| Poly(1-methylcyclohexyl 4-ethenylphenoxyacetate-4-hydroxystyrene) [Polymer of Synthesis Example 7(3)] | 6.0 g |
| 2-Methyl-2-p-toluenesulfonylpropiophenone (photoacid generator of Reference Example 4) | 0.3 g |
| Methyl lactate | 13.7 g |

As a result, the same good results as obtained in Examples 1 and 2 were obtained. A positive tone pattern was formed by using this resist material and showed an exposure energy amount of 15 mJ/cm$^2$.

Example 10

A resist material having the same composition was prepared and subjected to the same experiments as described in Examples 1 and 2.

| | |
|---|---|
| Poly(1-methylcyclopentyl 4-ethenylphenoxyacetate-4-hydroxystyrene) [Polymer of Synthesis Example 10] | 6.0 g |
| Bis(isopropylsulfonyl)diazomethane (photoacid generator of Reference Example 7) | 0.3 g |
| Diethylene glycol dimethyl ether | 13.7 g |

As a result, the same good results as obtained in Examples 1 and 2 were obtained. A positive tone pattern was formed by using this resist material and showed an exposure energy amount of 18 mJ/cm$^2$.

Example 11

A resist material having the same composition was prepared and subjected to the same experiments as described in Examples 1 and 2.

| | |
|---|---|
| Poly(2-tetrahydropyranyl 4-ethenylphenoxyacetate-4-hydroxystyrene) [Polymer of Synthesis Exmaple 9] | 6.0 g |
| 2-Nitrobenzyl trichloroacetate (photoacid generator of Reference Example 8) | 0.3 g |
| Diethylene glycol dimethyl ether | 13.7 g |

As a result, the same good results as obtained in Examples 1 and 2 were obtained. A positive tone pattern was formed by using this resist material and showed an exposure energy amount of 10 mJ/cm$^2$.

Comparative Examples 1 to 4

Resist materials having the compositions as listed in Table 3 were prepared.

Table 3

| | | |
|---|---|---|
| Comparative Example 1 | Poly(tert-butyl 4-ethenylphenoxy-acetate-4-hydroxystyrene) [Polymer of Synthesis Example 13] | 6.0 g |
| | 2-(Cyclohexylcarbonyl)-2-(p-toluenesulfonyl)propane (photoacid generator of Reference Example 1) | 0.3 g |
| | Diethylene glycol dimethyl ether | 13.7 g |
| Comparative Example 2 | Poly(4-tert-butoxystyrene-4-hydroxystyrene) | 6.0 g |
| | 2-(Cyclohexylcarbonyl)-2-(p-toluenesulfonyl)propane | 0.3 g |
| | Diethylene glycol dimethyl ether | 13.7 g |
| Comparative Example 3 | Poly(4-tetrahydropyranyloxy-styrene-4-hydroxystyrene) | 6.0 g |
| | 2-(Cyclohexylcarbonyl)-2-(p-toluenesulfonyl)propane | 0.3 g |
| | Diethylene glycol dimethyl ether | 13.7 g |
| Comparative Example 4 | Poly(4-tert-butoxycarbonyloxy-styrene-4-hydroxystyrene) | 6.0 g |
| | 2-(Cyclohexylcarbonyl)-2-(p-toluenesulfonyl)propane | 0.3 g |
| | Diethylene glycol dimethyl ether | 13.7 g |

The polymers used in Comparative Examples 2 to 4 were synthesized by the methods described in Japanese Patent Unexamined Publication No. 2-62544, European Patent Laid-Open Nos. 342,498 and 366,590.

Using the resist materials, the same experiments as described in Examples 1 and 2 were carried out. The results are shown in Table 4 and Figs. 3 and 4.

Table 4

| Comparative Example No. | Time from light exposure to baking | | |
|---|---|---|---|
| | 15 minutes | 30 minutes | 1 hour |
| 1 | Positive tone pattern with 0.4 μm L/S | Impossible to form pattern | - |
| 2 | Positive tone pattern with 0.4 μm L/S | Overhang | Impossible to form pattern |
| 3 | Positive tone pattern with 0.4 μm L/S | Impossible to form pattern | - |
| 4 | Positive tone pattern with 0.4 μm L/S | Impossible to form pattern | - |

Note) L/S: line and space.

As is clear from Table 4, and Figs. 3 and 4, when the treatment from the light exposure to baking takes 30 minutes to 1 hour, an overhang phenomenon as shown in Fig. 3 takes place or a pattern is not formed as shown in Fig. 4, wherein numeral 5a denotes a resin pattern.

As mentioned above, the resist material of the present invention has high transmittance for deep ultraviolet light, KrF excimer laser light, etc., high sensitivity for light exposure to these light sources, electron beams and X-ray radiation, is remarkably excellent in heat resistance and adhesion to the substrate, and able to form fine patterns with good shape of practically usable submicron order and able to maintain stable pattern dimensions with the lapse of time from the light exposure to heat treatment. Thus, the present invention has a large value for forming ultrafine pattern in semiconductor industry.

Further, the resist material of the present invention exhibits particularly good effects on forming patterns using deep ultraviolet light and KrF excimer laser, but is also applicable for pattern formation using i-line light, electron beams, X-rays, etc.

## Claims

**1.** A resist material comprising
(a) a polymer represented by the formula:

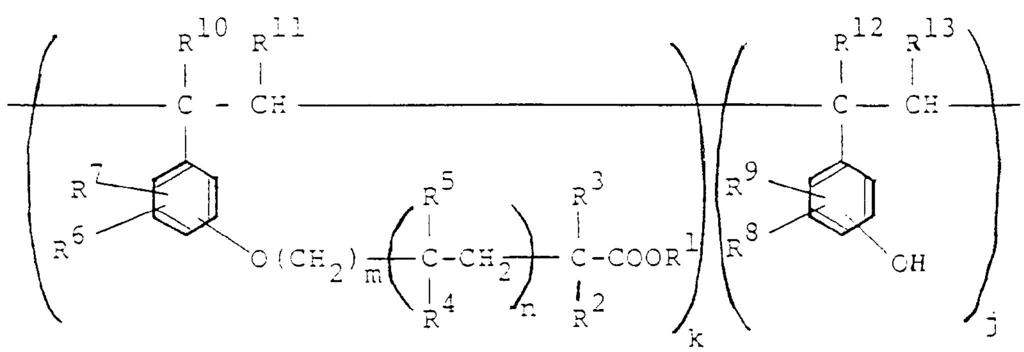

(1)

wherein $R^1$ is a tetrahydropyranyl group, a trimethylsilyl group, a group of the formula:

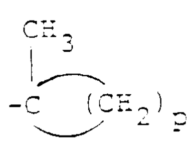

in which p is an integer of 4 or 5, or a group of the formula:

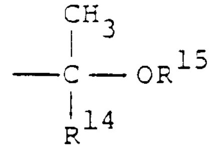

in which $R^{14}$ is a hydrogen atom, a methyl group or an ethyl group; $R^{15}$ is a straight-chain, branched or cyclic alkyl group having 1 to 6 carbon atoms; $R^2$ to $R^5$ are independently a hydrogen atom, or a straight-chain or branched alkyl group having 1 to 8 carbon atoms; $R^6$ to $R^{13}$ are independently a hydrogen atom, or a straight-chain or branched alkyl group having 1 to 6 carbon atoms; k and j are independently natural numbers, provided that k/k+j = 0.1 to 0.9; and m and n are independently zero or an integer of 1 to 3,

(b) a photo-sensitive compound capable of generating an acid when exposed to light, and
(c) a solvent for dissolving both the components (a) and (b)

**2.** A resist material according to Claim 1, wherein the polymer is represented by the formula:

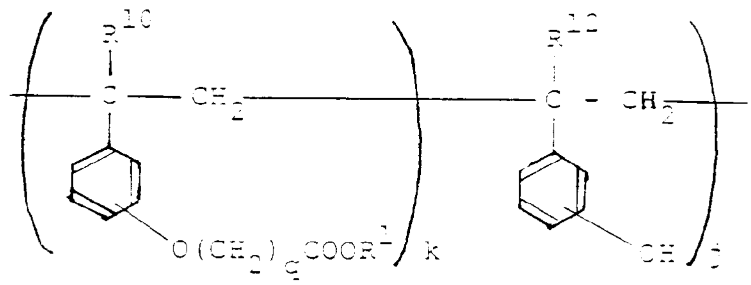

(2)

wherein q is an integer of 1 to 3; and $R^1$, $R^{10}$, $R^{12}$, k and j are as defined in Claim 1.

**3.** A resist material according to Claim 1, wherein the polymer is represented by the formula:

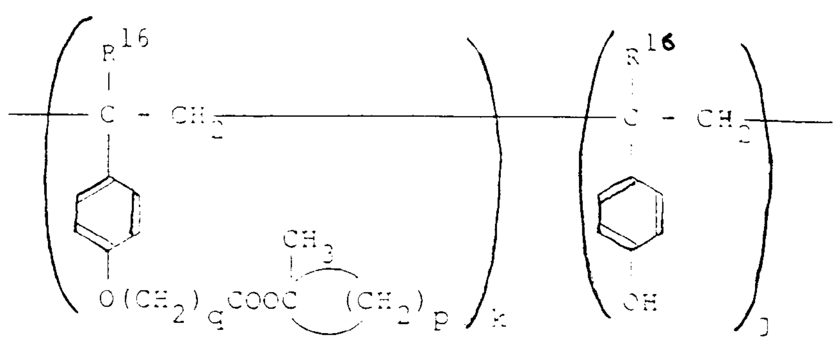

(3)

wherein $R^{16}$ is a hydrogen atom or a methyl group; q is an integer of 1 to 3; and p, k and j are as defined in Claim 1.

**4.** A resist material according to Claim 3, wherein the polymer of the formula (3) is a polymer of 1-methylcyclopentyl 4-ethenylphenoxyacetate and 4-hydroxystyrene, or a polymer of 1-methylcyclohexyl 4-ethenylphenoxyacetate and 4-hydroxystyrene.

**5.** A resist material according to Claim 1, wherein the polymer is represented by the formula:

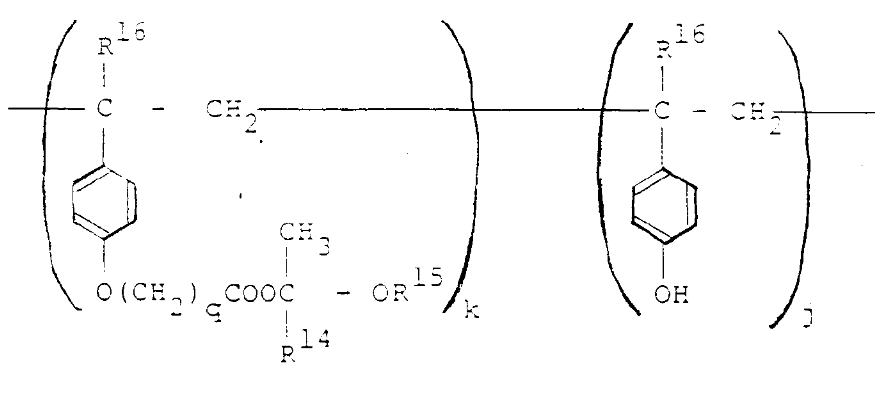

(4)

wherein $R^{16}$ is a hydrogen atom or a methyl group; q is an integer of 1 to 3; and $R^{14}$, $R^{15}$, k and j are as defined in Claim 1.

6. A resist material according to Claim 5, wherein the polymer of the formula (4) is a polymer of 1-cyclohexyloxyethyl 4-ethenylphenoxyacetate and 4-hydroxystyrene, a polymer of 1-methoxyethyl 4-ethenylphenoxyacetate and 4-hydroxystyrene, a polymer of 1-ethoxethyl 4-ethenylphenoxyacetate and 4-hydroxystyrene, a polymer of 1-methoxy-1-methylethyl 4-ethenylphenoxyacetate and 4-hydroxystyrene, a polymer of 1-methoxy-1-methylethyl 4-isopropenylphenoxyacetate and 4-hydroxy-a-methylstyrene, a polymer of 1-butoxyethyl 4-ethenylphenoxypropionate and 4-hydroxystyrene or a polymer of 1-isobutyloxyethyl 4-isopropenyl-phenoxyacetate and 4-hydroxy-a-methylstyrene.

7. A resist material according to Claim 1, wherein the photo-sensitive compound is represented by the formula:

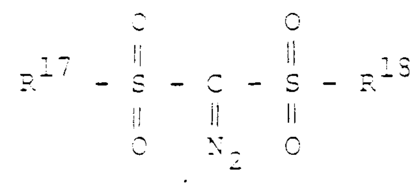

wherein $R^{17}$ and $R^{18}$ are independently a straight-chain, branched or cyclic alkyl group having 1 to 10 carbon atoms, a straight-chain or branched haloalkyl group having 1 to 10 carbon atoms, or a group of the formula:

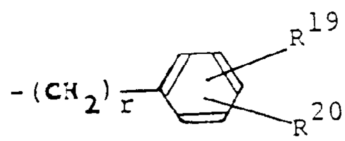

wherein $R^{19}$ and $R^{20}$ are independently a hydrogen atom, a halogen atom, a straight-chain, branched or cyclic alkyl group having 1 to 10 carbon atoms, a straight-chain or branched haloalkyl group, a straight-chain or branched alkoxy group, a nitro group or a cyano group; and r is zero or an integer of 1 to 5.

8. A resist material according to Claim 7, wherein in the formula (5), $R^{17}$ and $R^{18}$ are independently a straight-chain, branched or cyclic alkyl group having 1 to 10 carbon atoms, a phenyl group, an alkyl substituted phenyl group, a benzyl group, an alkyl substituted benzyl group, a phenethyl group, or an alkyl substituted phenethyl group.

9. A resist material according to Claim 7, wherein in the formula (5), $R^{17}$ and $R^{18}$ are independently a straight-chain, branched or cyclic alkyl group having 1 to 10 carbon atoms.

10. A resist material according to Claim 1, wherein the photo-sensitive compound is represented by the formula:

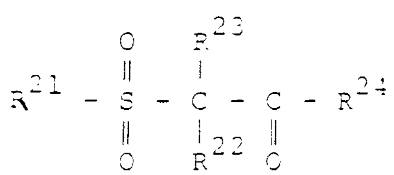 (6)

wherein $R^{21}$ is a straight-chain, branched or cyclic alkyl group having 1 to 10 carbon atoms, a trifluoromethyl group, a phenyl group or a p-tolyl group; $R^{22}$ is a hydrogen atom, a halogen atom, a straight-chain or branched alkyl group having 1 to 5 carbon atoms, or a straight-chain or branched haloalkyl group having 1 to 5 carbon atoms; $R^{23}$ is a hydrogen atom, a straight-chain or branched alkyl group having 1 to 5 carbon atoms, or a straight-chain or branched haloalkyl group having 1 to 5 carbon atoms; and $R^{24}$ is a

straight-chain branched or cyclic alkyl group having 1 to 10 carbon atoms, a phenyl group, a phenyl group substituted with a straight-chain or branched alkyl group having 1 to 5 carbon atoms, a straight-chain or branched alkoxy group, a halogen atom or an alkylthio group.

**11.** A resist material according to Claim 10, wherein in the formula (6) $R^{21}$ and $R^{24}$ are independently a straight-chain, branched or cyclic alkyl group having 1 to 10 carbon atoms, a phenyl group or a p-tolyl group; and $R^{22}$ and $R^{23}$ are methyl groups.

**12.** A resist material according to Claim 10, wherein in the formula (6) $R^{21}$ is a straight-chain, branched or cyclic alkyl group having 1 to 6 carbon atoms, a phenyl group or a p-tolyl group; $R^{24}$ is a branched or cyclic alkyl group having 3 to 6 carbon atoms, a phenyl group or a p-tolyl group; and $R^{22}$ and $R^{23}$ are methyl groups.

**13.** A resist material according to Claim 1, wherein the photo-sensitive compound is represented by the formula:

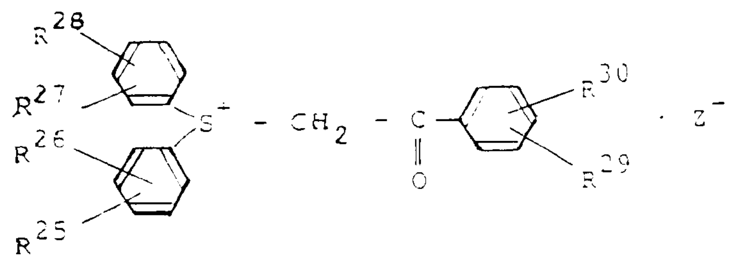

(7)

wherein $R^{25}$ to $R^{30}$ are independently a hydrogen atom, a halogen atom, a straight-chain, branched or cyclic alkyl group having 1 to 10 carbon atoms, or a straight-chain or branched alkoxy group; $Z^-$ is a perchlorate ion, a p-toluenesulfonate ion or a trifluoromethanesulfonate ion.

**14.** A resist material according to Claim 1, wherein the photo-sensitive compound is represented by the formula:

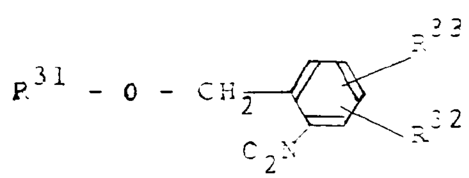

(8)

wherein $R^{31}$ is a trichloroacetyl group, a p-toluene-sulfonyl group, a p-trifluoromethylbenzene-sulfonyl group, a methanesulfonyl group or a trifluoromethanesulfonyl group; and $R^{32}$ and $R^{33}$ are independently a hydrogen atom, a halogen atom, or a nitro group.

**15.** A resist material according to any preceding claim further comprising (c) a solvent for dissolving both the components (a) and (b).

**16.** A process for forming a pattern which comprises

forming a film on a substrate using the resist material of claim 15,
exposing the film selectively to light,
heating the resulting film,
developing the film to remove exposed portions, and
forming a photoresist pattern on unexposed portions of the film.

**17.** A process according to Claim 16, wherein the light is i-line light , deep ultraiolet light, KrF excimer laser light, electron beams or X-rays.

FIG. 1A

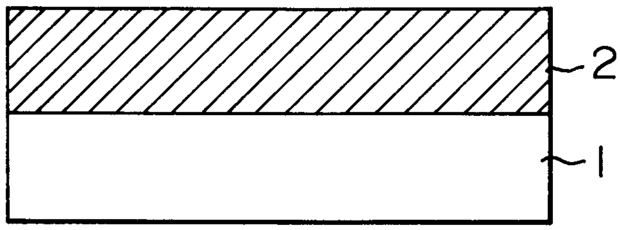


FIG. 1B

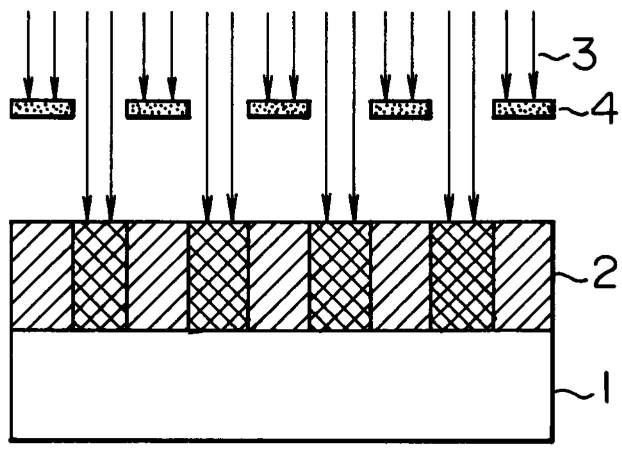


FIG. 1C

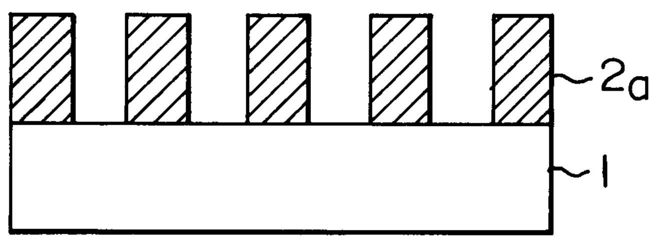

FIG. 2

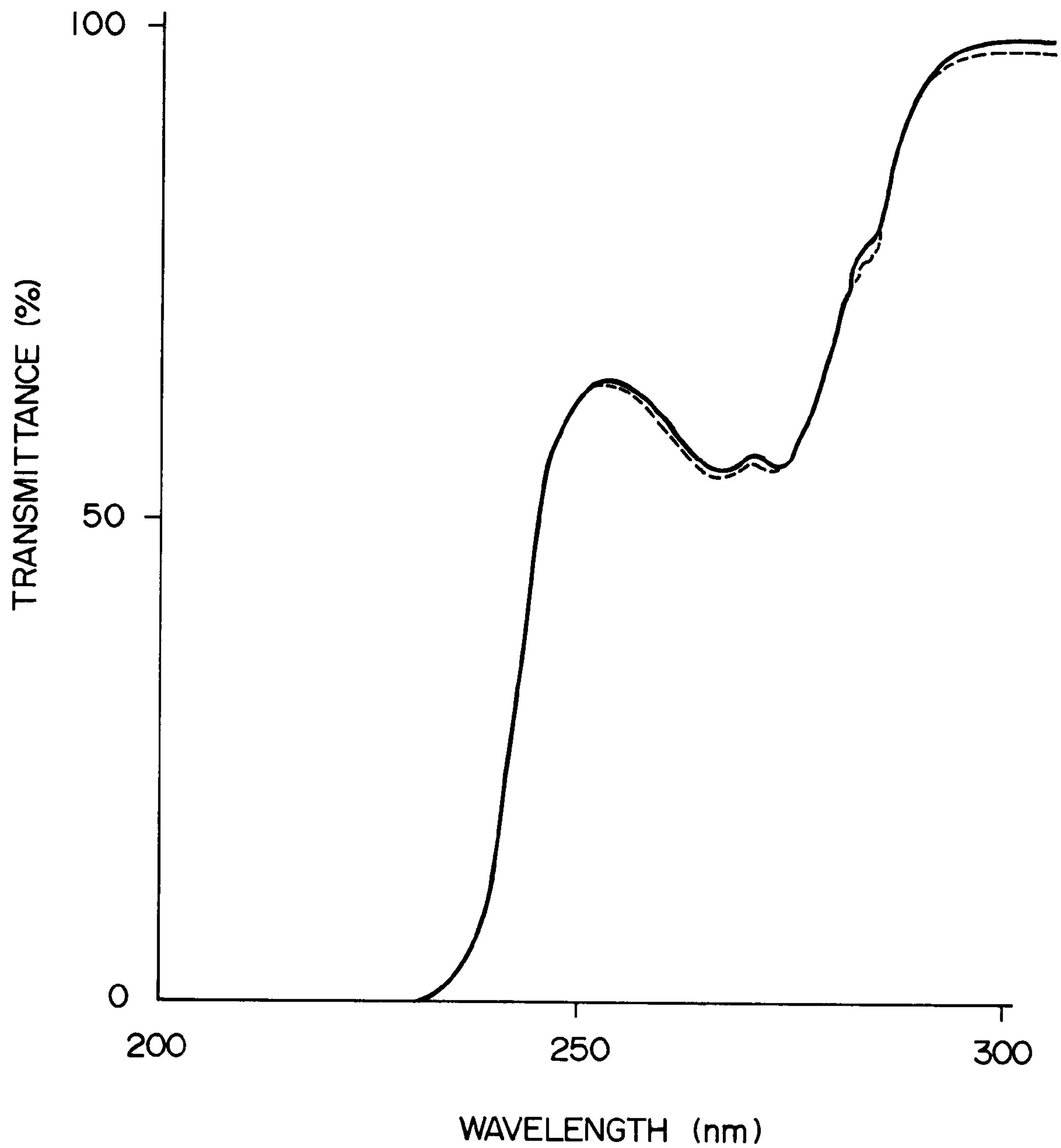

FIG. 3

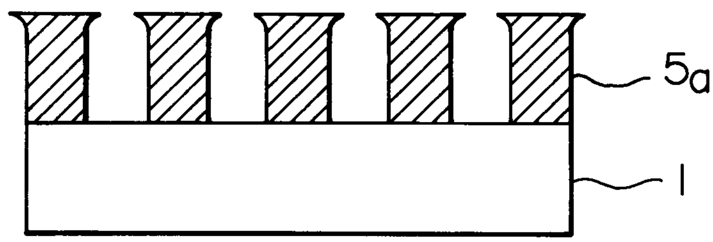


FIG. 4

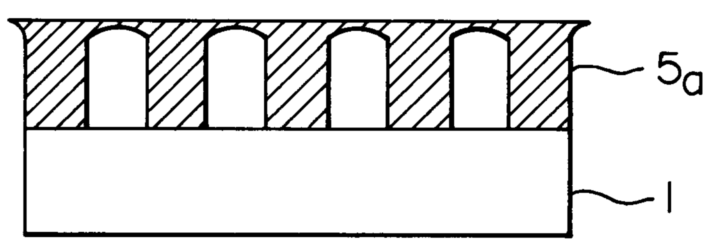

European Patent Office

# EUROPEAN SEARCH REPORT

Application Number

EP 91307908.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP - A - 0 366 590 (INTERNATIONAL BUSINESS MACHINES) * Claims 1-11 * -- | 1-6, 16,17 | G 03 F 7//039<br>G 03 F 7/075 |
| D,A | EP - A - 0 342 498 (BASF) * Claims * -- | 1-6, 13,16, 17 | |
| D,A | PATENT ABSTRACTS OF JAPAN, unexamined applications, section P, vol. 14, no. 240, May 22, 1990 THE PATENT OFFICE JAPANESE GOVERNEMENT page 127 P 1051 * Kokai-no. 2-62 544 (TOSOH CORP.) -- | 1-6, 16,17 | |
| D,A | US - A - 4 603 101 (CRIVELLO) * Claims * -- | 1-6, 13,16, 17 | TECHNICAL FIELDS SEARCHED (Int. Cl.5) |
| D,A | US - A - 4 491 628 (ITO) * Claims * ---- | 1-6, 13,16, 17 | G 03 F<br>H 05 K |

The present search report has been drawn up for all claims

| Place of search | Date of completion of the search | Examiner |
|---|---|---|
| VIENNA | 20-12-1991 | SCHÄFER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)